(19) Europäisches Patentamt
European Patent Office
Office européen des brevets

(11) Numéro de publication: **0 006 368**
**B1**

# (12) FASCICULE DE BREVET EUROPEEN

(45) Date de publication du fascicule de brevet: **18.11.81**

(51) Int. Cl.³: **C 07 D 277/46**, C 07 D 277/40, A 61 K 31/425

(21) Numéro de dépôt: **79400293.1**

(22) Date de dépôt: **09.05.79**

(54) **Dérivés de phényl-4 thiazolyl-2 oxamate, procédé pour leur préparation et leur application dans le traitement de l'asthme.**

(30) Priorité: **19.06.78 FR 7818500**

(43) Date de publication de la demande:
**09.01.80 Bulletin 80/1**

(45) Mention de la délivrance du brevet:
**18.11.81 Bulletin 81/46**

(84) Etats contractants désignés:
**AT BE CH DE GB IT LU NL SE**

(56) Documents cités:
**CH-A-486 481**

(73) Titulaire: **PIERRE FABRE S.A., 125, rue de la Faisanderie, F-75116 Paris (FR)**

(72) Inventeur: **Cousse, Henri, Foun de los Nobios Chemin de Lastinos, F-81100 Castres (FR)**
Inventeur: **Mouzin, Gilbert, 21, rue Sainte-Foy, F-81100 Castres (FR)**
Inventeur: **Tarayre, Jean-Pierre, Rue des Sports Valdurenque, F-81100 Castres (FR)**
Inventeur: **Casadio, Silvano, Via Tantardini, 15, I-20136 Milan (IT)**

(74) Mandataire: **Doat, Jean Pierre, 17, Avenue Jean Moulin, F-81106 Castres Cedex (FR)**

EP 0 006 368 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Dérivés de phényl-4 thiazolyl-2 oxamante, procédé pour leur préparation et leur application dans le traitement de l'asthme.

La présente invention, réalisée au Centre de Recherches Pierre Fabre, concerne de nouveaux dérivés de phényl-4 thiazolyl-2 oxamate, leur procédé de préparation et leur application en thérapeutique, notamment dans le traitement de l'asthme allergique et de la bronchite asthmatiforme avec composante allergique. L'invention vise également les compositions pharmaceutiques contenant ces principes actifs.

Les composés chimiques objet de l'invention répondent à la formule générale:

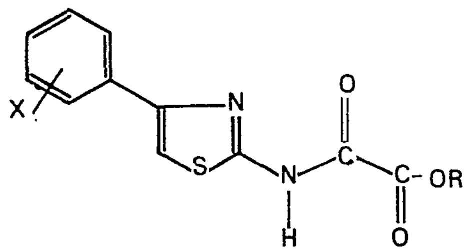

(I)

dans laquelle:

X qui se trouve en position 2, 3 ou 4 représente un hydrogène, un halogène, un méthyle ou un méthoxy.

R représente un groupe éthyle ou benzyle.

Les composés de formule générale (I) peuvent être préparés en deux étapes; par cyclisation d'un dérivé de l'acétophénone par la thiourée on obtient les amino-2 phényl-4 thiazoles, qui traités par un ester du chlorure d'oxalyle fournissent les dérivés (I) selon le schéma réactionnel:

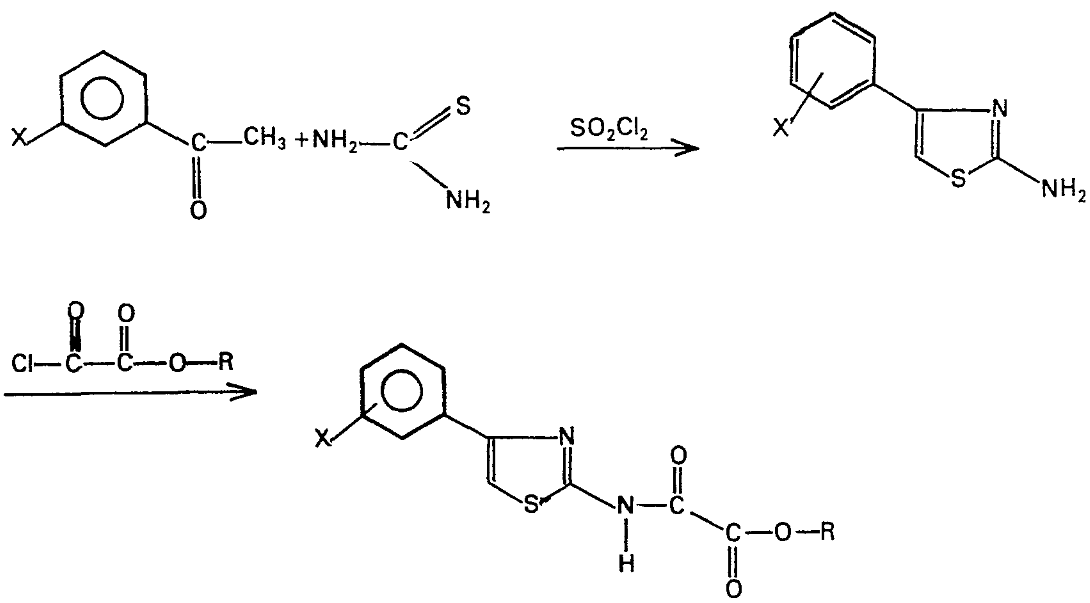


X et R ayant les significations données précédemment.

Les composés chimiques nouveaux suivants et leur mode de préparation sont cités à titre d'exemples non limitatifs.

Exemple 1:
(Phényl-4 thiazolyl-2) oxamate d'éthyle.
a) Phényl-4 amino-2 thiazole

Mélanger soigneusement 360 g (3 moles) d'acétophénone et 455 g (6 moles) de thiourée, puis ajouter par petites fractions 264 ml (3,3 moles) de chlorure de sulfuryle.

La réaction est exothermique et l'addition du chlorure de sulfuryl dure 2 heures.

Le milieu réactionnel se liquéfie puis se prend en masse, porter alors à 105 °C pendant 3 heures.

Laisser revenir à température ambiante, laver à l'acétone puis filtrer.

Les cristaux obtenus sont recristallisés dans 3 litres d'eau bouillante. On récupère le chlorhydrate de l'amine qui est traité par une solution d'ammoniaque à pH 12.

On récupère après filtration et séchage 412 g de produit (rendement 78%).

b) (phényl-4 thiazolyl-2) oxamate d'éthyle.

Une suspension de 168 g (2 moles) de bicarbonate de sodium dans une solution de 352 g (2 moles) de phényl-4 amino-2 thiazole et de 2,5 litres d'acétone est agitée à température ambiante.

Ajouter goutte à goutte 272 g (2 moles) de chloroglyoxylate d'éthyle et maintenir l'agitation une

nuit.

Après filtration, évaporer la phase cétonique jusqu'à siccité et le résidu jaune est recristallisé dans un mélange dioxanne - alcool.

On récupère 450 g de produit (rendement 75%) de formule:

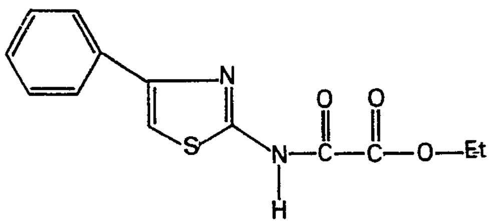

Formule brute: $C_{13}H_{12}N_2O_3S$
Masse moléculaire: 276,31
Cristaux jaunes
Point de fusion: 158 °C
Chromatographie en couche mince:
- support: gel de silice 60 F 254 Merck
- solvant: acide acétique, dioxanne, toluène 2/8/90
- révélation: UV et iode
- Rf: 0,60

Solubilités: Insoluble dans l'eau et dans l'éthanol. Soluble à 20% dans le diméthyl acétamide et à 25% dans la méthyl pyrrolidone.

Exemple 2:
(p-chlorophényl-4 thiazolyl-2) oxamate d'éthyle.
a) Para chloro phényl-4 amino-2 thiazole

D'une manière analogue à celle décrite dans l'exemple 1, mais en utilisant la parachloro acétophénone, on obtient le produit de formule:

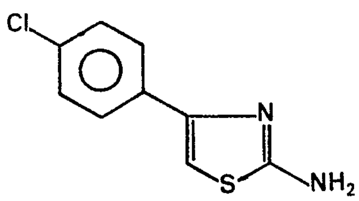

- Formule brute: $C_9H_7ClN_2S$
Masse moléculaire: 210,67
Cristaux: jaunes
Point de fusion: 167 °C
Chromatographie en couche mince:
- support: gel de silice 60 F 254 Merck
- solvant: acide acétique, dioxanne, toluène 2/8/90
- révélation: UV et iode
- Rf: 0,3

Solubilités: Insoluble dans l'eau. Soluble à 15% dans l'éthanol.

b) (p-chlorophényl-4 thiazolyl-2) oxamate d'éthyle.

Dans une solution glacée contenant 109,5 g d'amino-2 para chlorophényl-4 thiazole dans 2750 cm³ de tétrahydrofurane anhydre et 157 cm³ de triéthylamine, ajouter goutte à goutte sous forte agitation 73 cm³ de chloroglyoxylate d'éthyle en solution dans 50 cm³ de T.H.F. et maintenir 5 heures à température ambiante. Evaporer jusqu'à siccité la phase organique et laver à l'eau. On récupère avec un rendement de 95% 153,5 g de produit de formule:

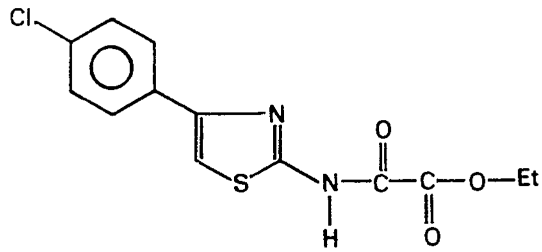

Formule brute: $C_{13}H_{11}ClN_2O_3S$
Masse moléculaire: 310,76
Cristaux jaunes
Point de fusion: 252 °C
Chromatographie en couche mince:
- support: gel de silice 60 F 254 Merck
- solvant: chloroforme - méthanol 95/5
- révélation: UV et iode
- Rf: 0,85

Solubilités: Insoluble dans l'eau et dans l'éthanol. Soluble dans le DMSO à 1% et à 4% dans la méthyl pyrrolidone.

Exemple 3:
(paraméthoxy phényl-4 thiazolyl-2) oxamate d'éthyle.
a) Paraméthoxy phényl-4 amino-2 thiazole

D'une manière analogue à celle décrite dans l'exemple 1, mais en utilisant la paraméthoxy acétophénone, on obtient le produit de formule:

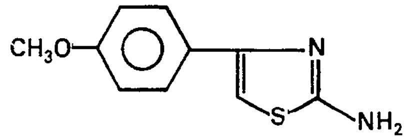

Formule brute: $C_{10}H_{10}N_2OS$
Masse moléculaire: 206,27
Cristaux jaunes
Point de fusion: 209 °C

b) (paraméthoxy phényl-4 thiazolyl-2) oxamate d'éthyle.

D'une manière analogue à celle décrite dans l'exemple 2, mais en utilisant le paraméthoxy phényl-4 amino-2 thiazole on obtient avec un rendement de 85% le produit de formule:

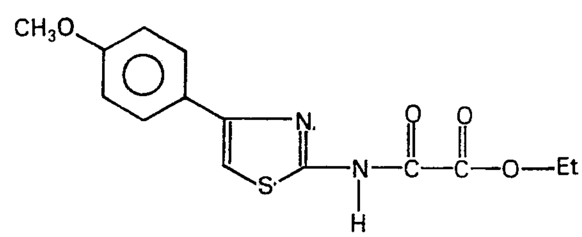

Formule brute: $C_{14}H_{14}N_2O_4S$
Masse moléculaire: 306,34
Cristaux beiges
Point de fusion: 168 °C
Chromatographie en couche mince:
- support: gel de silice 60 F 254 Merck
- solvant: toluène, dioxanne, acide acétique
- révélation: UV et iode
- Rf: 0,67

Solubilités: Insoluble dans l'eau et dans l'éthanol. Soluble dans le diméthyl sulfoxyde à 50% et la méthyl pyrrolidone à 50%.

Exemple 4:
(méta méthyl phényl-4 thiazolyl-2) oxamate de benzyle

D'une manière analogue à celle décrite précédemment mais en utilisant la méta méthyl acétophénone et le chloroglyoxylate de benzyle, on obtient le produit de formule:

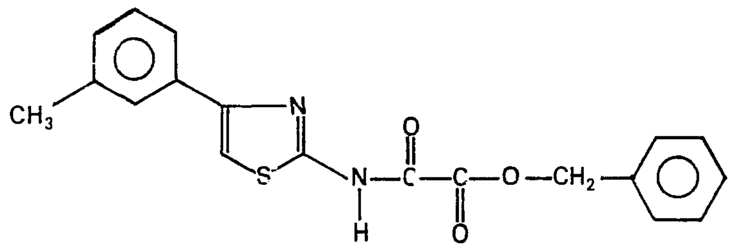

Exemple 5:
(Ortho bromo phényl-4 thiazolyl-2) oxamate de benzyle

D'une manière analogue à celle décrite dans les exemples précédents, mais en utilisant l'ortho bromo acétophénone et le chloroglyoxylate de benzyle on obtient le produit de formule:

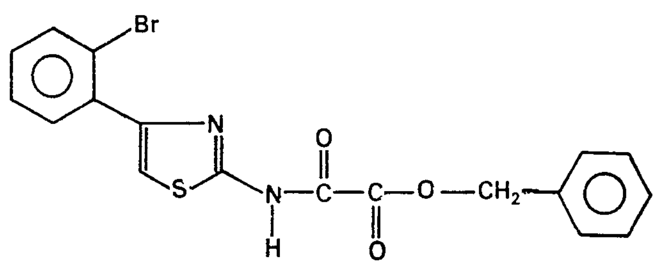

Expérimentations

Les composés chimiques précédemment décrits on fait l'objet d'expérimentations toxicologiques, pharmacologiques et cliniques en vue de déterminer les possibilités d'application dans le domaine thérapeutique.

1) Toxicologie (toxicité aigüe après une seule administration orale chez la souris; selon la méthode de Miller et Tainter (Proc. soc. exper. Biol. Med. 1944, 57, 261).

On administre les produits dans une suspension de Tween 80 + eau distillée. La mortalité est relevée au bout de 7 jours.
Le tableau ci-après récapitule les résultats obtenus:

| Produits | doses en mg/kg | nb souris par lot | mortalité au bout du 7ème j | DL 50 approximative mg/kg |
|---|---|---|---|---|
| Exemple 1 | 1000 | 10 | 1 | >1000 |
| Exemple 2 | 1000 | 10 | 0 | >1000 |
| Exemple 3 | 1000 | 10 | 1 | >1000 |
| Exemple 4 | 1000 | 10 | 1 | >1000 |
| Exemple 5 | 1000 | 10 | 2 | >1000 |

2) Pharmacodynamie:

Les résultats sont obtenus sur le test d'anaphylaxie passive cutanée selon la méthode de Brocklehurst W.E. in Handbook of experimental immunology, Ed. Weiz. D.M. Blackwell Scientific publications Oxford and Edimbourg, p. 745-751 (1967):

a) Préparer l'antisérum sur rats sensibilisés par l'antigène, l'injecter par voie intradermique.

b) Laisser un temps de latence de 24 à 48 h.

c) Administration du produit à tester
per os 10 minutes avant l'opération d)
i.v. simultanément à d).

d) Injection i.v. de l'antigène + colorant.

e) Sacrifier 30 minutes après et visualiser la réaction antigène-anticorps par la fuite du colorant due à l'augmentation de la perméabilité capillaire sous l'influence combinée de l'histamine et du

SRSA.

Cette action est exprimée en $ED_{50}$ dose qui réduit de moitié la fuite du colorant par rapport aux témoins.

A titre d'exemple non limitatifs les résultats de deux composés sont précisés:

| Produit | $ED_{50}$ mg/kg per os |
|---|---|
| Exemple 1 | 1 |
| Exemple 3 | 2 |

Les essais ont été effectués successivement à 30, 5, 1 et 0,2 mg/kg sur des lots homogènes de 10 rats compte tenu de leur insolubilité dans l'eau. Ces composés sont administrés en suspension dans le tween + eau.

3) Applications thérapeutiques

Compte tenu des propriétés pharmacologiques, ces composés et plus particulièrement les composés des exemples 1 et 3 sont utilisables par voie orale en préventif vis-à-vis des crises d'asthme allergiques.

Ces composés peuvent être utiles également dans le traitement de l'urticaire, des rhinites allergiques et certaines allergies cutanées.

A ces diverses fins, les composés de l'invention sont évidemment administrés en doses variant avec leur nature, avec le mode d'administration et avec le traitement recherché.

Pour l'homme la dose quotidienne totale peut être comprise entre 5 et 500 mg par jour.

**Revendications pour les Etats contractants:** BE, CH, DE, GB, IT, LU, NL, SE

1) Nouveaux dérivés de phényl-4 thiazolyl-2 oxamate répondant à la formule générale I:

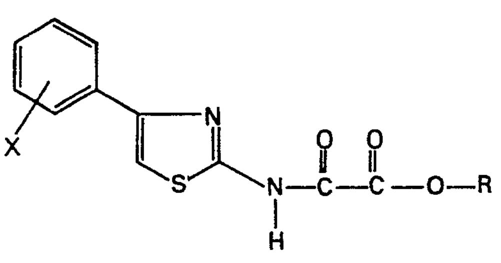
(I)

dans laquelle:
X qui se trouve en position 2, 3 ou 4 représente un hydrogène, un halogène, un méthyle ou un méthoxy.
R représente un groupe éthyle ou benzyle.

2) Composé de formule générale (I) selon la revendication 1: le (phényl-4 thiazolyl-2) oxamate d'éthyle.

3) Composé de formule générale (I) selon la revendication 1: le (p-chlorophényl-4 thiazolyl-2) oxamate d'éthyle.

4) Composé de formule générale (I) selon la revendication 1: le (p-méthoxyphényl-4 thiazolyl-2) oxamate d'éthyle.

5) Composé de formule générale (I) selon la revendication 1: le (m-méthylphényl-4 thiazolyl-2) oxamate de benzyle.

6) Composé de formule générale (I) selon la revendication 1: l'(o-bromo phényl-4 thiazolyl-2) oxamate de benzyle.

7) Procédé de préparation des composés de formule générale (I) selon les revendications 1 à 6, caractérisé par le fait qu'il consiste à faire réagir un phényl-4 amino-2 thiazole de formule générale (II):

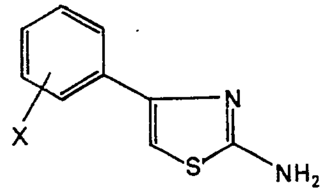
(II)

dans laquelle:
X a la signification donnée dans la revendication 1 avec un chloroglyoxylate de formule (III):

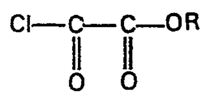
(III)

dans laquelle R a la signification donnée dans la revendication 1.

8) A titre de médicaments nouveaux utiles dans le traitement de l'asthme les composés selon les revendications 1 à 6.

**Revendications pour l'Etat contractant:** AT

1) Procédé de préparation des composés de formule générale (I):

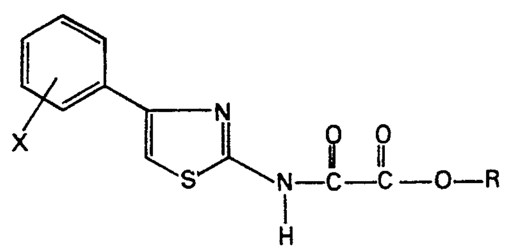
(I)

dans laquelle:
X qui se trouve en position 2, 3 ou 4 représente un hydrogène, un halogène, un méthyle ou un méthoxy.
R représente un groupe éthyle ou benzyle caractérisé par le fait qu'il consiste à faire réagir un phényl-4 amino-2 thiazole de formule générale II:

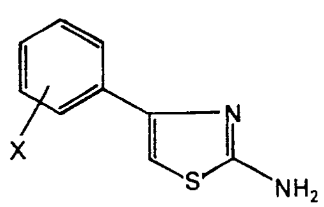
(II)

avec un chloroglyoxylate de formule III:

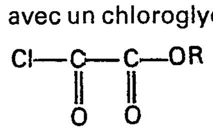
(III)

X et R ont la signification donnée pour les composés de formule générale I.

2) Procédé de préparation des composés de formule générale (I) selon la revendication 1, caractérisé en ce que l'on condense le phényl-4 amino-2 thiazole avec le chloroglyoxylate d'éthyle.

3) Procédé de préparation des composés de formule générale (I) selon la revendication 1, caractérisé en ce que l'on condense le parachlorophényle-4 amino-2 thiazole avec le chloroglyoxylate d'éthyle.

4) Procédé de préparation des composés de formule générale (I) selon la revendication 1, caractérisé en ce que l'on condense le paraméthoxy phényl-4 amino-2 thiazole avec le chloroglyoxylate d'éthyle.

5) Procédé de préparation des composés de formule générale (I) selon la revendication 1, caractérisé en ce que l'on condense le méta méthyl phényl-4 amino-2 thiazole avec le chloroglyoxylate de benzyle.

6) Procédé de préparation des composés de formule générale (I) selon la revendication 1, caractérisé en ce que l'on condense l'ortho bromo phényl-4 amino-2 thiazole avec le chloroglyoxylate de benzyle.

**Patentansprüche für die Vertragsstaaten:** BE, CH, DE, GB, IT, LU, NL, SE

1. Neue 4-Phenyl-2-thiazolyl-oxamat-Derivate der allgemeinen Formel I

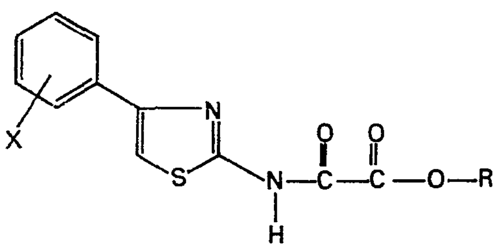

(I)

in der
X, das in der 2-, 3- oder 4-Stellung steht, ein Wasserstoffatom, ein Halogenatom, eine Methylgruppe oder eine Methoxygruppe und
R eine Äthylgruppe oder eine Benzylgruppe bedeuten.

2. Verbindung der allgemeinen Formel I nach Anspruch 1: (4-Phenyl-2-thiazolyl)-oxamsäureäthylester.

3. Verbindung der allgemeinen Formel I nach Anspruch 1: [4-(p-Chlorphenyl)-2-thiazolyl]-oxamsäureäthylester.

4. Verbindung der allgemeinen Formel I nach Anspruch 1: [4-(p-Methoxyphenyl)-2-thiazolyl]-oxamsäureäthylester.

5. Verbindung der allgemeinen Formel I nach Anspruch 1: [4-(m-Methylphenyl)-2-thiazolyl]-oxamsäurebenzylester.

6. Verbindung der allgemeinen Formel I nach Anspruch 1: [4-(o-Bromphenyl)-2-thiazolyl]-oxamsäurebenzylester.

7. Verfahren zur Herstellung der Verbindungen der allgemeinen Formel I nach den Ansprüchen 1 bis 6, dadurch gekennzeichnet, dass man ein 4-Phenyl-2-amino-thiazol der allgemeinen Formel II

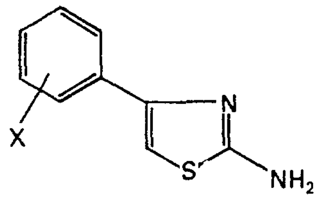

(II)

in der X die in Anspruch 1 angegebenen Bedeutungen besitzt,
mit einem Chlorglyoxylat der allgemeinen Formel III

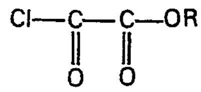

(III)

in der R die in Anspruch 1 angegebenen Bedeutungen besitzt,
umsetzt.

8. Arzneimittel zur Behandlung von Asthma, enthaltend die Verbindungen nach den Ansprüchen 1 bis 6.

**Patentansprüche für den Vertragsstaat:** AT

1. Verfahren zur Herstellung von 4-Phenyl-2-thiazolyl-oxamat-Derivaten der allgemeinen Formel I,

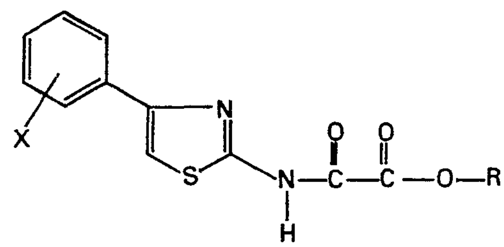

(I)

in der
X, das in der 2-, 3- oder 4-Stellung steht, ein Wasserstoffatom, ein Halogenatom, eine Methylgruppe oder eine Methoxygruppe und
R eine Äthylgruppe oder eine Benzylgruppe bedeuten, dadurch gekennzeichnet, dass man ein 4-Phenyl-2-amino-thiazol der allgemeinen Formel II

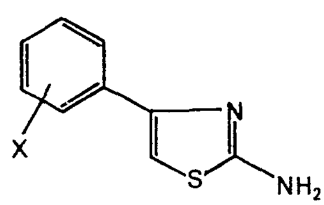

(II)

mit einem Chlorglyoxylat der allgemeinen Formel III,

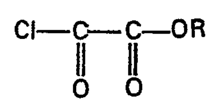

(III)

in der X und R die für die Verbindungen der allgemeinen Formel I angegebenen Bedeutungen besitzen, umsetzt.

2. Verfahren zur Herstellung der Verbindungen der allgemeinen Formel I nach Anspruch 1, dadurch gekennzeichnet, dass man 4-Phenyl-2-amino-thiazol mit Chlorglyoxylsäureäthylester kondensiert.

3. Verfahren zur Herstellung der Verbindungen der allgemeinen Formel I nach Anspruch 1, dadurch gekennzeichnet, dass man 4-(p-Chlorphenyl)-2-amino-thiazol mit Chlorglyoxylsäureäthylester kondensiert.

4. Verfahren zur Herstellung der Verbindungen der allgemeinen Formel I nach Anspruch 1, dadurch gekennzeichnet, dass man 4-(p-Methoxyphenyl)-2-amino-thiazol mit Chlorglyoxylsäureäthylester kondensiert.

5. Verfahren zur Herstellung der Verbindungen der allgemeinen Formel I nach Anspruch 1, dadurch gekennzeichnet, dass man 4-(m-Methylphenyl)-2-amino-thiazol mit Chlorglyoxylsäurebenzylester kondensiert.

6. Verfahren zur Herstellung der Verbindungen der allgemeinen Formel I nach Anspruch 1, dadurch gekennzeichnet, dass man 4-(o-Bromphenyl)-2-amino-thiazol mit Chlorglyoxylsäurebenzylester kondensiert.

**Claims for the Contracting States:** BE, CH, DE, GB, IT, LU, NL, SE

1) Novel derivatives of 4-phenyl-2-thiazolyl oxamate corresponding to the general formula I

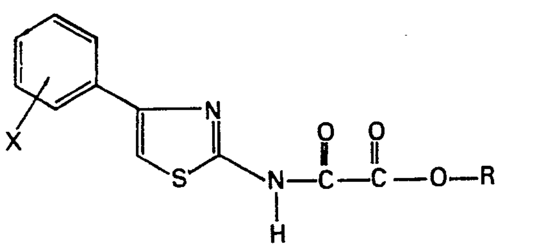

(I)

in which
X, which is in the 2-, 3- or 4-position, represents hydrogen, halogen, methyl or methoxy, and.
R represents an ethyl or benzyl group.

2) Compound of the general formula (I) according to claim 1: (4-phenyl-2-thiazolyl)ethyl oxamate.

3) Compound of the general formula (I) according to claim 1: (4-p-chlorophenyl-2-thiazolyl)ethyl oxamate.

4) Compound of the general formula (I) according to claim 1: (4-p-methoxyphenyl-2-thiazolyl)ethyl oxamate.

5) Compound of the general formula (I) according to claim 1: (4-m-methylphenyl-2-thiazolyl)benzyl oxamate.

6) Compound of the general formula (I) according to claim 1: (4-o-bromophenyl-2-thiazolyl)benzyl oxamate.

7) Process for the preparation of compounds of the general formula (I) according to claims 1 to 6, characterised in that a 4-phenyl-2-aminothiazole of the general formula (II)

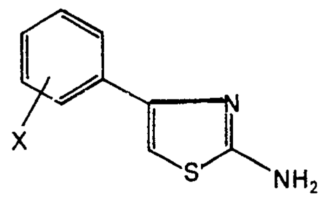

(II)

in which
X has the meaning given in claim 1
is reacted with a chloroglyoxylate of the formula (III)

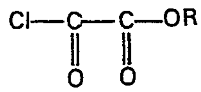

in which
R has the meaning given in claim 1.

8) The compounds according to claims 1 to 6 as novel medicaments for use in the treatment of asthma.

**Claims for the Contracting State:** AT

1) Process for the preparation of compounds of the general formula (I)

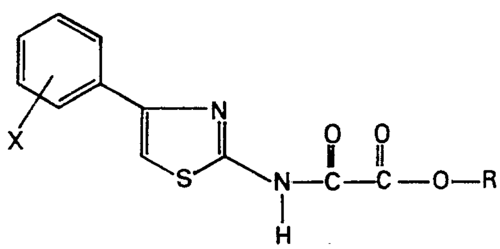

(I)

in which
X, which is in the 2-, 3- or 4-position, represents hydrogen, halogen, methyl or methoxy, and
R represents an ethyl or benzyl group,
characterised in that a 4-phenyl-2-aminothiazole of the general formula (II)

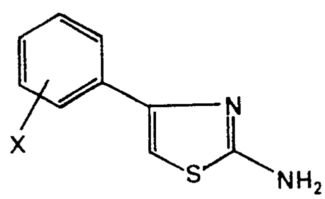

(II)

is reacted with a chloroglyoxylate of the formula (III)

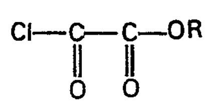

X and R have meaning given for the compounds of the general formula I.

2) Process for the preparation of the compounds of the general formula (I) according to claim 1, characterised in that 4-phenyl-2-amino-

thiazole is condensed with ethyl chloroglyoxylate.

3) Process for the preparation of the compounds of the general formula (I) according to claim 1, characterised in that 4-para-chlorophenyl-2-aminothiazole is condensed with ethyl chloroglyoxylate.

4) Process for the preparation of the compounds of the general formula (I) according to claim 1, characterised in that 4-para-methoxyphenyl-2-aminothiazole is condensed in ethyl chloroglyoxylate.

5) Process for the preparation of the compounds of the general formula (I) according to claim 1, characterised in that 4-meta-methylphenyl-2-aminothiazole is condensed with benzyl chloroglyoxylate.

6) Process for the preparation of the compounds of the general formula (I) according to claim 1, characterised in that 4-ortho-bromophenyl-2-aminothiazole is condensed with benzyl chloroglyoxylate.